# EUROPEAN PATENT SPECIFICATION

(11) **EP 2 457 496 B1**
(45) Date of publication and mention of the grant of the patent: **08.01.2014**
(21) Application number: 11189308.7
(22) Date of filing: 16.11.2011
(51) Int. Cl.: A61B 1/06, C09J 1/00

(54) **Illumination optical unit for endoscope, method of manufacturing the same, and adhesive for endoscope optical member**
Optische Beleuchtungseinheit für Endoskop, Herstellungsverfahren dafür und Haftstoff für ein optisches Endoskopelement
Unité optique d'éclairage pour endoscope, son procédé de fabrication et adhésif pour élément optique d'endoscope

(30) Priority: 25.11.2010 JP 2010262604; 19.10.2011 JP 2011229767
(43) Date of publication of application: 30.05.2012
(73) Proprietor: Fujifilm Corporation, Minato-ku Tokyo 106-8620 (JP)
(72) Inventor: Komukai, Makito, Ashigarakami-gun Kanagawa, 258-8538 (JP); Mizuyoshi, Akira, Ashigarakami-gun Kanagawa, 258-8538 (JP)
(74) Representative: Klunker . Schmitt-Nilson . Hirsch

(56) References cited:
- EP-A1- 0 097 225
- EP-A1- 1 795 798
- FR-A1- 2 698 876
- US-A- 4 866 108
- US-A1- 2010 172 148

## Description

### BACKGROUND OF THE INVENTION

**1. Field of the Invention**

The present invention relates to an illumination optical unit for an endoscope that irradiates a part to be observed in a subject with illumination light in order to observe the inside of the subject, a method of manufacturing the same, and an adhesive for an endoscope optical member.

**2. Description of the Related Art**

In the related art, diagnosis using an endoscope is widely prevalent in the medical field. The endoscope includes, at the distal end of an insertion part to be inserted into a subject, an observation window for incorporating image light of the subject and an illumination window for radiating illumination light toward the subject. The endoscope is connected to a light source device via a cord or a connector.

The light source device has a light source for supplying the illumination light for illuminating the inside of the subject to the endoscope. The illumination light from the light source is guided to the distal end of the insertion part by a light guide inserted through the endoscope. In the related art, a white light source, such as a xenon lamp or a halogen lamp, has been used as the light source that constitutes the light source device. However, a light source device using a laser beam source has recently began to be used instead of this white light source. An endoscope in which a laser beam supplied from the light source device using this laser beam source is guided to the distal end of the insertion part by the light guide, and a fluorescent body arranged at the distal end of the light guide is excited by the laser beam to emit light so as to irradiate a body cavity with white illumination light is described in JP2007-20937A.

Additionally, the endoscope is required to radiate a higher intensity of illumination light. Therefore, a reflective film with high reflectivity may be provided around the fluorescent body in order to efficiently use the excitation emission light as the illumination light. As this reflective film with high reflectivity, it is known that a metal film of silver, aluminum, or the like is suitable.
Documents US 2010/0172148 A1 and EP 1 795 798 A1 discloses optical units according to the preamble of claim 1.

### SUMMARY OF THE INVENTION

When diagnosis is performed using an endoscope, the inside of the endoscope insertion part to be inserted into a body cavity is brought into a high-humidity state, and grease containing molybdenum disulfide is applied to the outer peripheral face of the insertion part as a lubricant. Moreover, the endoscope is subjected to a washing disinfection process of dipping the endoscope in a disinfectant including peracetic acid or the like after the end of diagnosis. As such, since moisture, grease, and medicine such as a disinfectant is apt to enter the inside of the endoscope insertion part, the endoscope is in an environment in which the fluorescent body or the reflective film that is vulnerable to moisture or medicine is apt to deteriorate.

Thus, the present inventor has studied the illumination optical unit for an endoscope of the structure in which the outer periphery of the fluorescent body is covered with a cylindrical sleeve member, and the distal end of the sleeve member is sealed with a protective cover that transmits illumination light. In the sealing of the protective cover to the sleeve member, an adhesive should be disposed between the protective cover and the sleeve member to firmly bond the protective cover and the sleeve member together.

However, among adhesives that bond the protective cover and the sleeve member together, there is an adhesive having low viscosity. If the low-viscosity adhesive is used, there is a possibility that the adhesive may pass through near the inner peripheral face of the sleeve member and the outer peripheral face of the protective cover and may flow in to the position where the fluorescent body should be originally arranged. Particularly when the illumination optical unit for an endoscope is manufactured, in a case where a step of inserting the fluorescent body into the sleeve is performed after passing through a step of bonding the protective cover and the sleeve member together, the solidified adhesive interferes with the insertion of the fluorescent body and a gap is formed between the protective cover and the fluorescent body, if the adhesive flows in to the position where the fluorescent body should be originally arranged. Additionally, even in a case where the step of bonding the protective cover and the sleeve is performed after the step of inserting the fluorescent body into the sleeve, the adhesive enters between the fluorescent body and the protective cover to form a gap therebetween if the adhesive flows too much. If volatilized gas enters the gap formed between the protective cover and the fluorescent body when diagnosis is performed using the endoscope or when washing disinfection processing, the fluorescent body and the reflective film will deteriorate and the intensity of illumination light will decrease.

The present invention has been made in view of the above-mentioned problems and an object of the present invention is to provide preventing an adhesive disposed between a protective cover and a sleeve member from flowing in to a position where a fluorescent body should be originally arranged.

An illumination optical unit for an endoscope of the present invention includes: an optical fiber that guides a laser beam supplied from a laser beam source to the distal end of the optical fiber and emits the guided laser beam from the distal end; a fluorescent body that is excited by the laser beam emitted from the optical fiber to emit florescent light and that forms white light including the fluorescent light and the laser beam; a sleeve member that covers the outer periphery of the fluorescent body and has the distal end opened; and a protective cover that is held by the sleeve member, covers the distal end side of the fluorescent body, and transmits the fluorescent light and the laser beam. Here, an adhesive having glass beads mixed thereinto is disposed at a gap between the sleeve member and the protective cover to seal the distal end of the sleeve member.

Preferably, the sleeve member is formed with a receiving portion including an inner peripheral face that faces the outer peripheral face of the protective cover and a bottom face that intersects the inner peripheral face and faces an end face of the protective cover, and the adhesive is disposed at least at a gap between the outer peripheral face and the inner peripheral face.

Preferably, the glass beads to be mixed into the adhesive are smaller than the distance between the outer peripheral face and the inner peripheral face. Additionally, preferably, the average particle size of the glass beads is 0.4 to 0.7 times the distance between the outer peripheral face and the inner peripheral face. Moreover, preferably, the particle size of the glass beads is equal to or greater than 3.2 µm and less than 20 µm. Furthermore, preferably, the bottom face of the sleeve member is formed with a circumferential groove.

Preferably, the illumination optical unit for an endoscope further includes a holding member having a fluorescent body holding portion that holds the fluorescent body and is opened on the distal end side that faces the protective cover, and a through hole that is continuous with the proximal end of the fluorescent body holding portion and allows the optical fiber to be inserted therethrough. The holding member is formed in a substantially cylindrical shape that fits to the sleeve member, and the surface of the fluorescent body holding portion is provided with a reflective film that reflects the white light emitted from the fluorescent body.

Preferably, the adhesive is a silicon-based adhesive. Preferably, glass beads to be mixed into the adhesive have a light-scattering property.

A method of manufacturing an illumination optical unit for an endoscope of the present invention is a method of manufacturing an illumination optical unit for an endoscope including: an optical fiber that guides a laser beam supplied from a laser beam source to the distal end of the optical fiber and emits the guided laser beam from the distal end; a fluorescent body that is excited by the laser beam emitted from the optical fiber to emit florescent light and that forms white light including the fluorescent light and the laser beam; a sleeve member that covers the outer periphery of the fluorescent body and has the distal end opened; and a protective cover that is held by the sleeve member, covers the distal end side of the fluorescent body, and transmits the fluorescent light and the laser beam. The method includes: disposing an adhesive having glass beads mixed thereinto at least at a gap between the sleeve member and the protective cover to seal the distal end of the sleeve member with the protective cover; inserting the fluorescent body and the optical fiber into the inside of the sleeve member from the proximal end side of the sleeve member whose distal end is sealed with the protective cover to bring the fluorescent body into close contact with the protective cover; and making the sleeve hold the fluorescent body and the optical fiber in a state where the fluorescent body is brought into close contact with the protective cover, and the optical fiber is arranged on the proximal end side of the fluorescent body.

According to the present invention, since the adhesive having the glass beads mixed thereinto is disposed at the gap between to sleeve member and the protective cover to seal the distal end of the sleeve member that covers the outer periphery of the fluorescent body, the excessive flow of the adhesive can be suppressed to prevent the adhesive from flowing in to the position where the fluorescent body should be arranged.

### BRIEF DESCRIPTION OF THE DRAWINGS

Fig. 1 is an outline view showing the configuration of an electronic endoscope system.

Fig. 2 is a cross-sectional view of essential portions showing the configuration of a distal end portion of an electronic endoscope.

Fig. 3 is a plan view of the distal end portion of the electronic endoscope.

Fig. 4 is a block diagram showing an electrical configuration of the electronic endoscope system.

Fig. 5 is an exploded perspective view showing the configuration of an illumination optical unit.

Fig. 6 is a cross-sectional view of essential portions showing a configuration around a fluorescent body.

Fig. 7 is a distribution map showing an example of the particle size distribution of glass beads to be mixed into an adhesive.

Fig. 8 is a cross-sectional view of essential portions showing a modification in which a receiving portion is formed with a groove.

### DESCRIPTION OF THE PREFERRED EMBODIMENTS

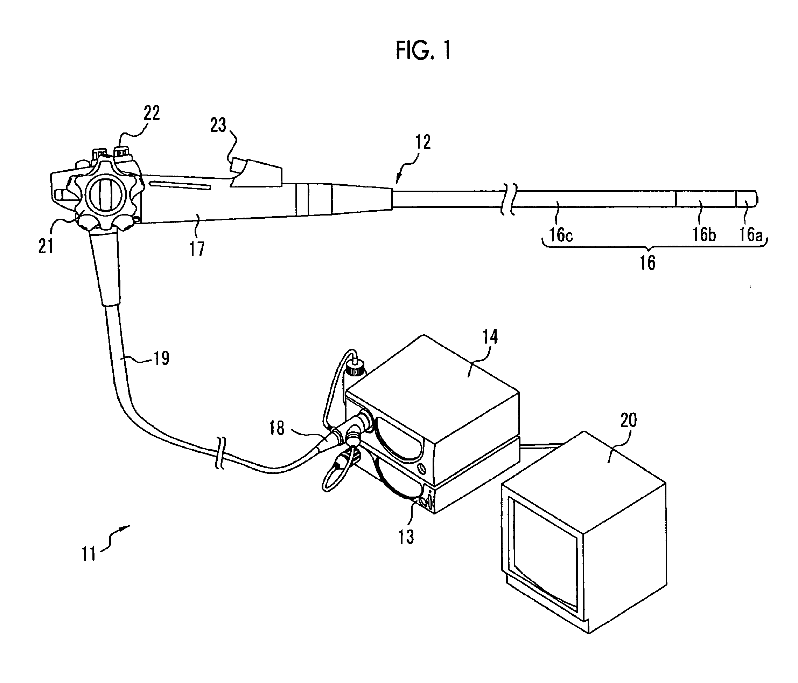

As shown in Fig. 1, an electronic endoscope system 11 includes an electronic endoscope 12, a processor device 13, and a light source device 14. The electronic endoscope 12 has a flexible insertion part 16 to be inserted into the body of a subject, a manipulating part 17 connected to a proximal end portion of the insertion part 16, a connector 18 connected to the processor device 13 and the light source device 14, and a universal cord 19 connecting the manipulation part 17 and the connector 18 together.

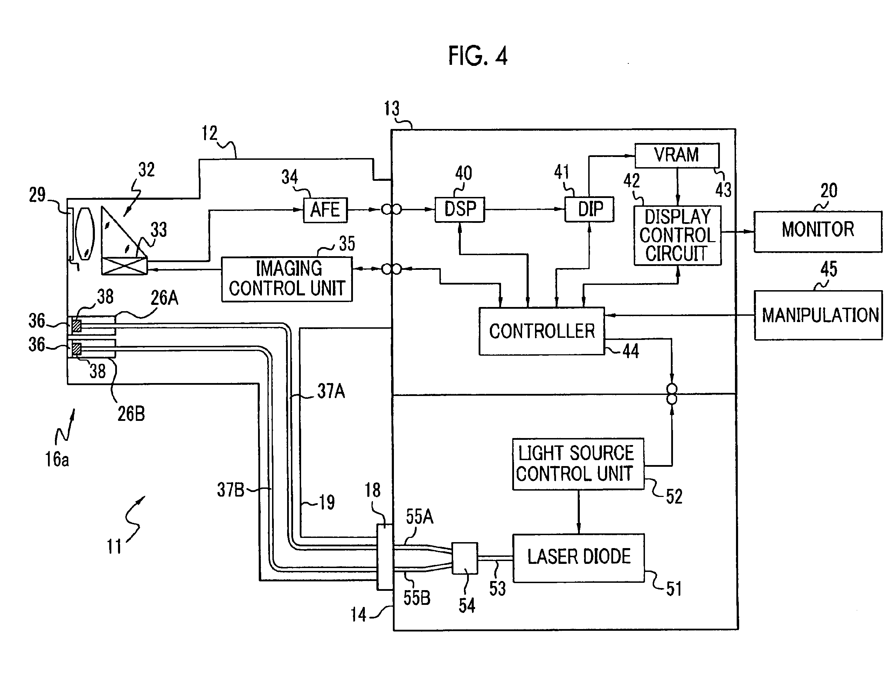

The insertion part 16 includes a distal end portion 16a provided at the distal end thereof and having a CCD type image sensor 33 (refer to Fig. 4, hereinafter referred to as CCD) for radiographing the inside of a subject built therein, a bendable bending portion 16b connected to the proximal end of the distal end portion 16a, and a flexible tube portion 16c connected to the proximal end of the bending portion 16b and having flexibility.

The manipulating part 17 is provided with manipulating members, such as an angle knob 21 for bending the bending portion 16b vertically and horizontally, and air supply and water supply buttons 22 for jetting air and water from the distal end portion 16a. Additionally, the manipulating part 17 is provided with a forceps opening 23 for allowing treatment tools, such as an electric scalpel, to be inserted into a forceps channel (not shown).

The processor device 13 is electrically connected to the light source device 14, and controls the operation of the electronic endoscope system 11 in general. The processor device 13 supplies electric power to the electronic endoscope 12 via a transmission cable inserted through the universal cord 19 or the insertion part 16, and controls the driving of the CCD 33. Additionally, the processor device 13 acquires an imaging signal output from the CCD 33 via the transmission cable, and performs various image processing to generate image data. The image data generated by the processor device 13 is displayed as an observation image on a monitor 20 cable-connected to the processor device 13.

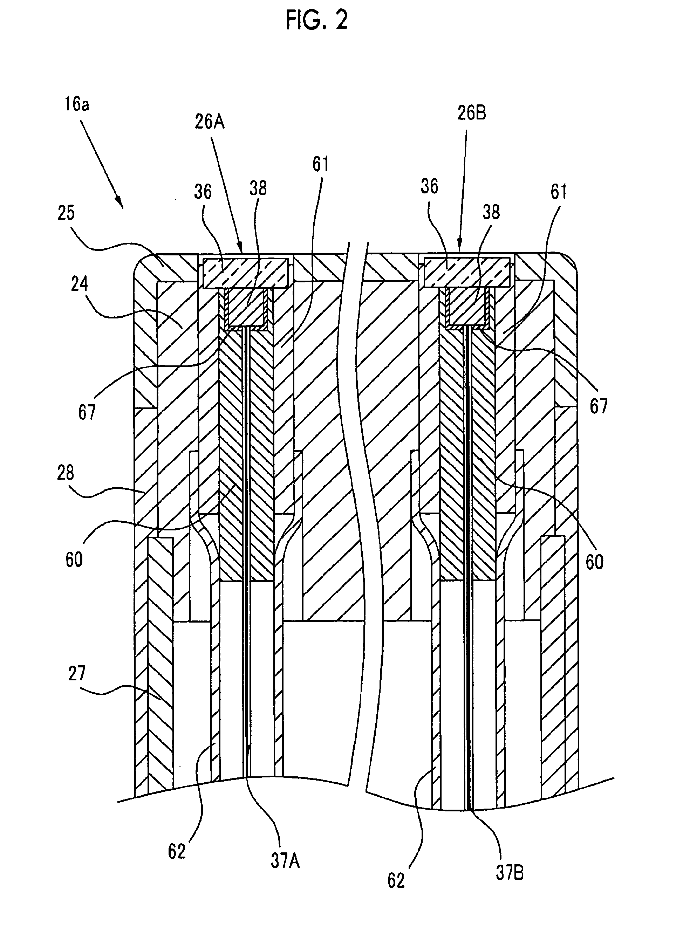

As shown in Fig. 2, the distal end portion 16a includes a distal end hard section 24, and a distal end protection cap 25 mounted on the distal end face of the distal end hard section 24. The distal end hard section 24 is made of metal, such as stainless steel, and has a plurality of through holes formed along the longitudinal direction thereof. Various parts, such as an imaging optical system 32 (refer to Fig. 4), the CCD 33, illumination optical units 26A and 26B, the forceps channel, and the air supply and water supply channels (not shown), are attached to the respective through holes of the distal end hard section 24. The rear end of the distal end hard section 24 is coupled to a bending piece 27 at the distal end that constitutes the bending portion 16b. Additionally, the outer periphery of the distal end hard section 24 is covered with a jacket tube 28.

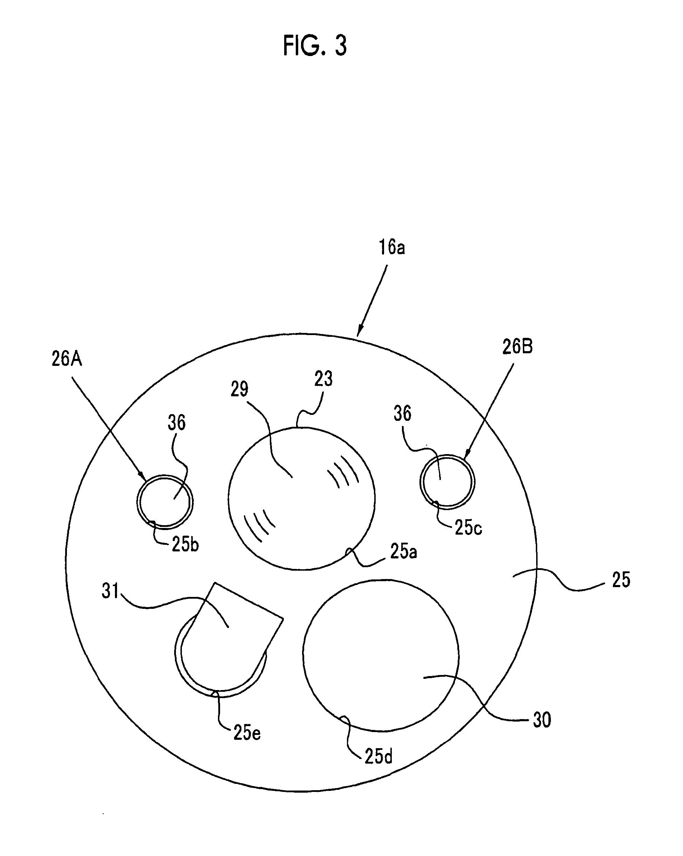

The distal end protection cap 25 is made of rubber or resin, and is formed with through holes at positions corresponding to the various parts held by the distal end hard section 24. As shown in Fig. 3, the distal end protection cap 25 exposes an observation window 29, the illumination optical units 26A and 26B, a forceps outlet 30, an air supply and water supply nozzle 31, and the like from through holes 25a to 25e. A pair of illumination optical units 26A and 26B is disposed at positions symmetrical to each other across the observation window 29.

As shown in Fig. 4, the CCD 33 is arranged in an inner part of the observation window 29 inside the distal end portion 16a such that an image in a subject is focused on an imaging plane by the imaging optical system 32 including a lens group and a prism.

The CCD 33 performs photoelectric conversion of the image in the subject focused on the imaging plane by the imaging optical system 32 to accumulate signal charges, and outputs the accumulated signal charges as imaging signals. The output imaging signals are sent to an AFE 34. The AFE 34 is constituted by a correlated double sampling (CDS) circuit, an automatic gain control (AGC) circuit, an A/D converter (all of these are not shown), and the like. The CDS performs correlated double sampling processing on the imaging signals output from the CCD 33, and removes the noise generated by driving the CCD 33. The AGC amplifies the imaging signals whose noise is removed by the CDS.

The imaging control unit 35 is connected to a controller 44 in the processor device 13 when the electronic endoscope 12 and the processor device 13 are connected together, and sends a driving signal to the CCD 33 when an instruction is issued from the controller 44. The CCD 33 outputs the imaging signals to the AFE 34 at a predetermined frame rate on the basis of the driving signal from the imaging control unit 35.

The illumination optical units 26A and 26B are units that radiate illumination light into a subject. The distal end face of the illumination optical units 26A and 26B is sealed by a protective cover 36, and is exposed from the distal end face of the distal end portion 16a, that is, the through holes 25b and 25c of the distal end protection cap 25, as an illumination window.

Optical fibers 37A and 37B that constitute the illumination optical units 26A and 26B guide a blue laser beam supplied from the light source device 14, and emit the laser beam to the fluorescent body 38 provided on the emission end side. Hereinafter, the emission end side of the optical fibers 37A and 37B is referred to as "distal end side", and the incident end side of the optical fibers 37A and 37B is referred to as "proximal end side". The fluorescent body 38 is made of, for example, YAG or BAM (BaMgAl₁₀O₁₇), absorbs portions of the blue laser beam emitted from the optical fibers 37A and 37B and is excited to emit green to yellow light beams. For this reason, in the illumination optical units 26A and 26B, a blue light beam transmitted through the fluorescent body 38 while being diffused in the fluorescent body and the green to yellow fluorescent light beams excited and emitted from the fluorescent body 38 are combined together to form white (quasi-white) illumination light.
The irradiation range of the illumination light is approximately equal to or greater than a radiographing range using the electronic endoscope 12, and the illumination light is almost uniformly radiated to the entire surface of an observation image.

The processor device 13 includes a digital signal processing circuit (DSP) 40, a digital image processing circuit (DIP) 41, a display control circuit 42, a VRAM 43, a controller 44, a manipulation unit 45, and the like.

The controller 44 controls the operation of the processor device 13 in general. The DSP 40 performs various signal processing, such as color separation, color interpolation, gain compensation, white balance adjustment, and gamma compensation, on the imaging signals output from the AFE 34 of the electronic endoscope 12, and generates image data. The image data generated by the DSP 40 is input to a working memory of a DIP 41. Additionally, the DSP 40 generates ALC control data required for the automatic control (ALC control) of, for example, the quantity of illumination light, such as an average luminance value obtained by averaging the luminance of respective pixels of the image data generated, and inputs the ALC control data to the controller 44.

The DIP 41 performs various image processing, such as electronic variable magnification, color enhancement processing, and edge enhancement processing, on the image data generated by the DSP 40. The image data subjected the various image processing by the DIP 41 is temporarily stored in a VRAM 43 as an observation image, and input to the display control circuit 42. The display control circuit 42 selects and acquires the observation image from the VRAM 43, and displays the observation image on the monitor 20.

The manipulation unit 45 includes well-known input devices, such as a control panel, a mouse, and a keyboard that are provided in the housing of the processor device 13. The controller 44 operates respective parts of the electronic endoscope system 11 according to a manipulation signal from the manipulation unit 45 or the manipulating part 17 of the electronic endoscope 12.

The light source device 14 includes a laser diode (LD) 51 and a light source control unit 52 as a laser beam source. The LD 51 is a light source that emits a blue laser beam with a central wavelength of 445 nm and that guides the laser beam to an optical fiber 53 via a condensing lens (not shown). The optical fiber 53 is connected to two optical fibers 55A and 55B via a branch coupler 54. The optical fibers 55A and 55B are connected to the optical fibers 37A and 37B of the electronic endoscope 12 via a connector 18. For this reason, a blue laser beam emitted from LD 51 is incident on the fluorescent body 38 that constitutes the illumination optical units 26A and 26B. Then, due to the blue laser light being made incident, the green to yellow fluorescent light beams excited and emitted from the fluorescent body 38 are combined therewith and radiated into a subject as white (quasi-white) illumination light.

The light source control unit 52 adjusts the timing of turning-on and turning-off of the LD 51 according to a control signal or synchronization signal input from the controller 44 of the processor device 13. Moreover, the light source control unit 52 communicates with the controller 44 and adjusts the quantity of light emission of the LD 51, thereby adjusting the quantity of the illumination light radiated into a subject. The control of the quantity of illumination light by the light source control unit 52 is ALC (Auto Light Control) control that adjusts the quantity of illumination light automatically according to the luminance of the radiographed observation image or the like, and is performed on the basis of the ALC control data generated by the DSP 40.

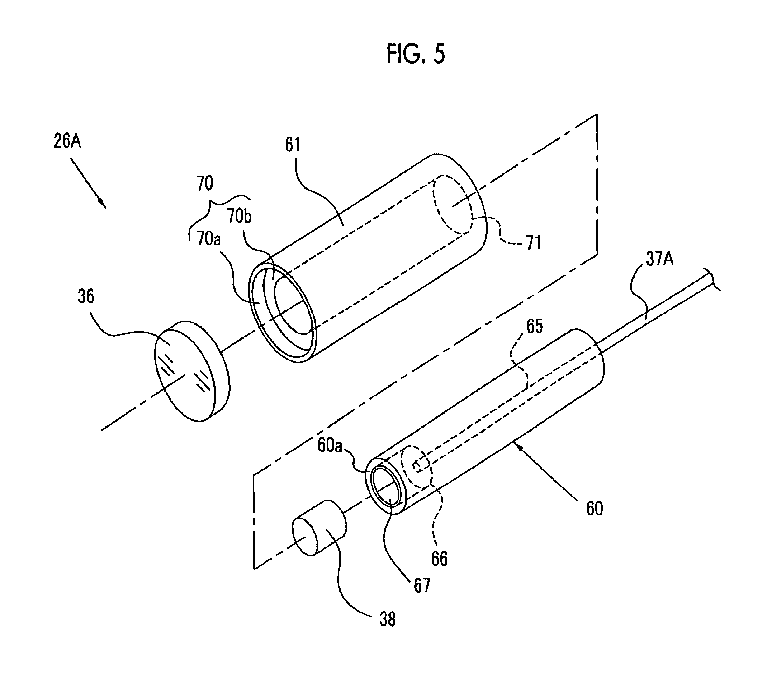

As shown in Figs. 2 and 5, the illumination optical unit 26A includes the single mode optical fiber 37A, the fluorescent body 38, a ferrule 60 as a holding member holding the fluorescent body 38 and the optical fiber 37A, a tubular sleeve member 61 that covers the outer periphery of the fluorescent body 38, and the protective cover 36 that seals the distal end of the sleeve member 61. Additionally, the illumination optical unit 26B includes the optical fiber 37B, the fluorescent body 38, the ferrule 60, the sleeve member 61, and the protective cover 36, and similarly to the illumination optical unit 26A, has a configuration in which the ferrule 60 holds the fluorescent body 38 and the optical fiber 37B, the sleeve member 61 covers the outer periphery of the fluorescent body 38, and the protective cover 36 seals the distal end of the sleeve member 61. Additionally, outer peripheral faces of the optical fibers 37A and 37B are covered with a protective tube 62 (refer to Fig. 2). A distal end portion of the protective tube 62 is fixed to the outer peripheral face of the sleeve member 61.

The ferrule 60 is formed in a substantially cylindrical shape, and has an insertion hole 65 through which the optical fiber 37A is inserted. A fluorescent body holding portion 66 that holds the fluorescent body 38 is formed on the distal end side of the ferrule 60. The fluorescent body holding portion 66 becomes concave from a distal end face 60a of the ferrule 60 in conformity with the appearance of the fluorescent body 38, and is formed in the shape of a recess that is opened on the distal end side that faces the protective cover 36. The insertion hole 65 is continuous with the proximal end of the fluorescent body holding portion 66.

The surface of the fluorescent body holding portion 66 is provided with a reflective film 67. The reflective film 67 is made of a metal film, such as silver or aluminum, and is formed in the shape of a thin film, for example, by plating, vapor deposition, sputtering, or the like. The fluorescent body 38 is held inside the fluorescent body holding portion 66 in contact with the reflective film 67. The illumination light emitted from the fluorescent body 38 can be reflected by the reflective film 67 and efficiently used. When the fluorescent body 38 is held by the fluorescent body holding portion 66, the distal end faces of the fluorescent body 38 and the reflective film 67 are formed so as to be flush with the distal end face 60a of the ferrule 60. The insertion hole 65 is formed along the central axis of the ferrule 60. The distal end portion of the optical fiber 37A fits into the insertion hole 65, and is held behind the fluorescent body 38.

The sleeve member 61 is formed in a substantially cylindrical shape that has a receiving portion 70 that receives the protective cover 36, and a fitting hole 71 into which the outer peripheral face 60b of the ferrule 60 fits, sequentially from the distal end side. The receiving portion 70 is formed so as to have a larger internal diameter than that of the fitting hole 71. The receiving portion 70 has an inner peripheral face 70a that faces the outer peripheral face 36a of the protective cover 36, and a bottom face 70b that intersects the inner peripheral face 70a and faces a proximal end face 36b of the protective cover 36. As the protective cover 36 is bonded to the receiving portion 70, the distal end of the sleeve member 61 is sealed. The fitting hole 71 is continuous up to a rear end face of the sleeve member 61 along the center of the sleeve member 61 from the bottom face 70b.

The protective cover 36 is formed substantially in the shape of a disc from a material through which the illumination light (white light) emitted from the fluorescent body 38, that is, the blue light beam transmitted through the fluorescent body 38 while being diffused in the fluorescent body and the green to yellow fluorescent light beam excited and emitted from the fluorescent body 38 can be transmitted. The protective cover 36 is formed from, for example, quartz glass, sapphire glass, or the like.

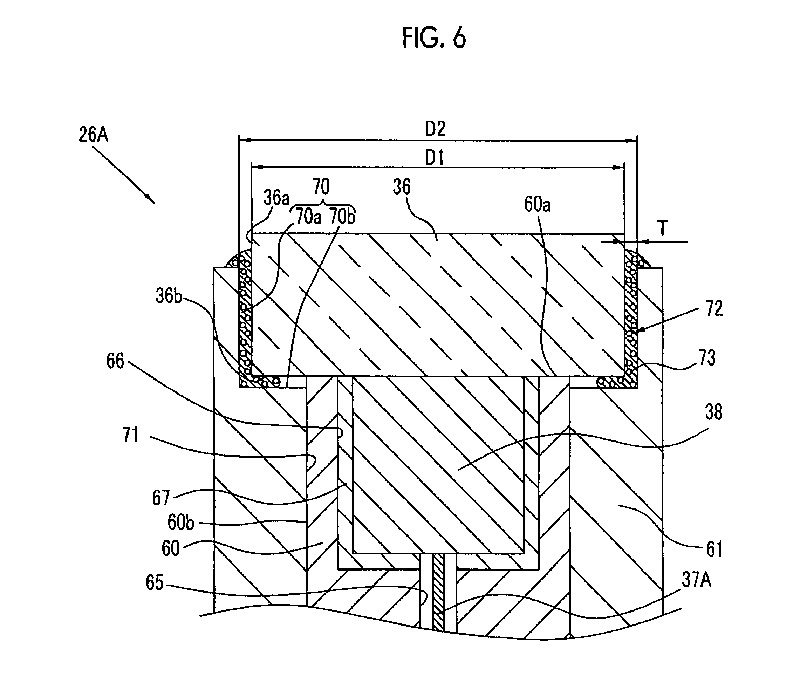

As shown in Fig. 6, when the protective cover 36 is bonded to the receiving portion 70 in order to seal the distal end of the sleeve member 61, an adhesive 72 is disposed at a gap between the receiving portion 70 and the protective cover 36, that is, between the inner peripheral face 70a of the receiving portion 70 and the outer peripheral face 36a of the protective cover 36. Specifically, an adhesive may be applied, poured, or sprayed between the inner peripheral face 70a of the receiving portion 70 and the outer peripheral face 36a of the protective cover 36. Otherwise, a beltlike member (not shown) on which an adhesive is applied may be affixed. As an adhesive 72 to be used for the bonding between the protective cover 36 and the receiving portion 70, for example, a silicon-based adhesive is used.

When the viscosity of the adhesive 72 to be poured into the gap between the protective cover 36 and the receiving portion 70 is low, the adhesive 72 may flow excessively and the adhesive 72 may flow into the inside of the fitting hole 71 that is a place where the fluorescent body 38 is arranged. Thus, in order to suppress the excessive flow of the adhesive 72, in the present embodiment, glass beads 73 are mixed into the adhesive 72. As the glass beads 73 to be mixed into the adhesive 72, glass beads having a light-scattering property are used. If the adhesive 72 into which the glass beads 73 are mixed is used, the adhesive 72 into which the glass beads 73 are mixed enter a gap T between the outer peripheral face 36a of the protective cover 36 and the inner peripheral face 70a of the receiving portion 70 so as to fill the gap. Therefore, the excessive flow of the adhesive 72 is suppressed, and the adhesive 72 is prevented from flowing into the fitting hole 71. At this time, the adhesive 72 is desirably disposed at least between the outer peripheral face 36a of the protective cover 36 and the inner peripheral face 70a of the receiving portion 70, and may be disposed between the proximal end face 36b of the protective cover 36 and the facing bottom face 70b. Additionally, since the adhesive 72 into which the glass beads 73 are mixed is seen in white, a form when the adhesive 72 flows in can be visually recognized easily. In addition, the ratio of the glass beads 73 to be mixed into the adhesive 72 is appropriately determined depending on the viscosity of the adhesive 72.

As the glass beads 73 to be mixed into the adhesive 72, glass beads having a particle size R customized to the gap T formed between the protective cover 36 and the receiving portion 70 are used. If the external diameter (the diameter of the outer peripheral face 36a) of the protective cover 36 is D1, and the internal diameter (the diameter of the inner peripheral face 70a) of the receiving portion 70 is D2, the gap T formed between the inner peripheral face 70a of the receiving portion 70 and the outer peripheral face 36a of the protective cover 36 becomes a portion on one side of the difference (D2-D1) between the internal diameter D2 and the external diameter D1, that is, (D2-D1)/2. The glass beads 73 of the particle size R suitable for this gap T= (D2-D1)/2 are used. Thereby, when the protective cover 36 is bonded to the receiving portion 70 in order to seal the distal end of the sleeve member 61, if the adhesive 72 into which the glass beads 73 of the above-described particle size R are mixed is poured between the inner peripheral face 70a and the outer peripheral face 36a, the glass beads 73 enter the gap T formed between the inner peripheral face 70a and the outer peripheral face 36a so as to fill the gap.

Moreover, if the adhesive 72 into which the glass beads 73 are mixed is poured into the gap T and the bonding of the protective cover 36 to the receiving portion 70 is performed, the whole peripheral face of the protective cover 36 is surrounded by the glass beads 73 that have entered the gap T so as to fill the gap. Hence, the protective cover 36 can be moved so as to be automatically pushed to the center of the receiving portion 70 to perform centering.

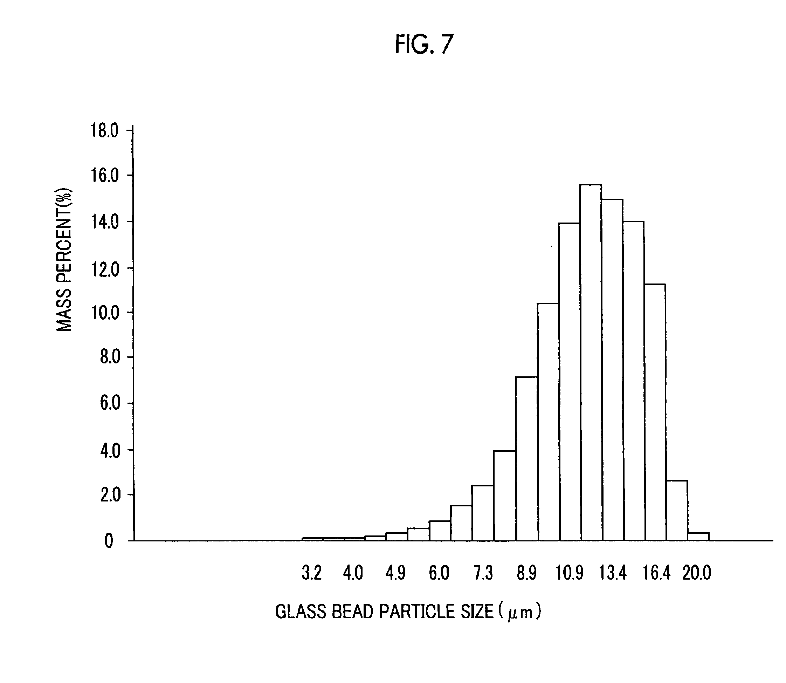

When a specific example is taken, in a case where the design difference (D2-D1) between the internal diameter D2 of the receiving portion 70 and the external diameter D1 of the protective cover 36 is 20 to 60 µm (value including intersection), the gap T=(D2-D1)/2 is 10-30 µm, and the central value is 20 µm. Thus, commercially available glass beads 73 with the particle size R matched to this gap T are selected. Fig. 7 shows an example of the particle size distribution included in the commercially available glass beads 73 selected in order to match the above-described gap T. In this example, glass beads 73 with a particle size R of 3.2 µm or more and less than 20 µm are included, and the central value of the gap T that is the distance between the inner peripheral face 70a and the outer peripheral face 36a is smaller than 20 µm. Moreover, a number of glass beads 73 of 12 µm that are the average value of the particle size R are included. If the commercially available glass beads 73 having the distribution of the particle size R matched to such a gap T are mixed into the adhesive 72, it is possible to suppress the excessive flow of the adhesive 72, and the centering of the protective cover 36 with respect to the receiving portion 70 is performed with high precision. In addition, the glass beads 73 to be mixed into the adhesive 72 are not limited to the example of the above-described particle size distribution, and may have the particle size R that is smaller than the average dimension of the gap T formed between the inner peripheral face 70a of the receiving portion 70 and the outer peripheral face 36a of the protective cover 36. In this case, if the ratio of the average value of the particle size R and the gap T is too large, the number of glass beads with the particle size R that do not enter into the gap T increases, and if the ratio is too small, degradation of the viscosity and degradation of the centering function are caused. Therefore, the average value of the particle size R is preferably 0.4 to 0.7 times the gap T.

In a manufacturing process of manufacturing the illumination optical unit 26A of the above configuration, first, a bonding step of bonding the protective cover 36 to the receiving portion 70 is performed in order to seal the distal end of the sleeve member 61. In this bonding step, a predetermined amount of the adhesive 72 into which the glass beads 73 are mixed is poured into and cured in the gap T formed between the inner peripheral face 70a of the receiving portion 70 and the outer peripheral face 36a of the protective cover 36. After this bonding step, the ferrule 60 that holds the fluorescent body 38 and the optical fiber 37A is inserted into the inside of the sleeve member 61 to bring the fluorescent body 38, the ferrule 60, and the reflective film 67 into close contact with the protective cover 36. Then, the sleeve member 61 is made to hold the ferrule 60 in a state where the ferrule is inserted to a position where the fluorescent body 38, the ferrule 60, and the reflective film 67 are brought into close contact with the protective cover 36. As the configuration in which the sleeve member 61 is made to hold the ferrule 60, the ferrule 60 can be fixed by the sleeve member 61 when the ferrule is pushed by setting the fitting between the fitting hole 71 and the outer peripheral face 60b of the ferrule 60 becomes tight fitting. In addition, the present invention is not limited to this, and the sleeve member 61 may be made to hold the ferrule 60 with means, such as bonding or screw fixing.

As described above, since the adhesive 72 into which the glass beads 73 are mixed is poured to bond the protective cover 36 to the receiving portion 70 of the sleeve member 61, the adhesive 72 can be prevented from flowing into the fitting hole 71. Thereby, even if the adhesive is cured, the fluorescent body 38, the ferrule 60, and the reflective film 67 can be brought into close contact with the protective cover 36 without hindering the insertion of the ferrule 60 into the fitting hole 71. Thus, when diagnosis using the electronic endoscope 12, washing disinfection processing, or the like is performed, it is possible to prevent gas from advancing between the fluorescent body 38 and the protective cover 36 to prevent the fluorescent body 38 and the reflective film 67 from deteriorating.

Additionally, since the glass beads 73 to be mixed into the adhesive 72 have a light-scattering property, when illumination light is radiated into a subject from the illumination optical units 26A and 26B, the glass beads 73 that have entered the gap T formed between the inner peripheral face 70a of the receiving portion 70 and the outer peripheral face 36a of the protective cover 36 scatter and reflect the illumination light emitted from the fluorescent body 38. This can suppress the uneven illumination of the illumination light radiated into a subject. When a transparent adhesive is used as in the related-art illumination optical unit, the illumination light emitted from the fluorescent body is transmitted through the protective cover and the adhesive and reflected by the inner peripheral face of the sleeve, whereby uneven illumination is apt to occur. However, in the present embodiment, there is no such uneven illumination because the adhesive 72 into which the glass beads 73 are mixed is used.

Additionally, since the adhesive 72 that is seen in white by making the glass beads 73 mixed into the adhesive able to be easily visually recognized, a worker can confirm whether the adhesive 72 is flowing so as to fill the gap T formed between the inner peripheral face 70a of the receiving portion 70 and the outer peripheral face 36a of the protective cover 36. Furthermore when a silicon-based adhesive that does not contain a sulfur component that oxidizes and sulfurizes the reflective film 67 is used for the bonding between the sleeve member 61 and the protective cover 36, deterioration of the reflective film 67 can be further prevented.

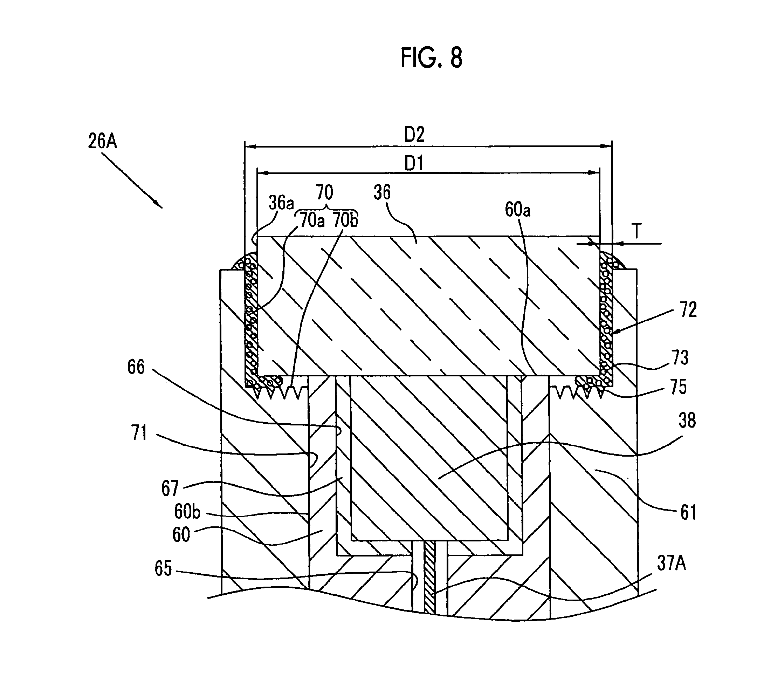

Although the receiving portion 70 of the sleeve member 61 that receives the protective cover 36 is formed in a smooth face in the above embodiment, the present invention is not limited thereto, and a groove may be formed in a face that constitutes the receiving portion 70. An example in which the receiving portion 70 is formed with a groove 75 is shown in Fig. 8. In this case, the bottom face 70b of the receiving portion 70 is formed with a circumferential groove 75 that is arranged concentrically with the inner peripheral face 70a. In addition, the shape of the groove 75 is not limited to the circumferential shape, and the groove may be spirally formed, for example. The adhesive 72 into which the glass beads 73 are mixed, which is the same as the above embodiment, is used for the bonding between the sleeve member 61 provided with such a groove 75, and the protective cover 36. When the adhesive 72 into which the glass beads 73 are mixed is poured between the inner peripheral face 70a of the receiving portion 70 and the outer peripheral face 36a of the protective cover 36, the adhesive 72 that has entered the bottom face 70b is apt to flow along the groove 75. Therefore, the excessive flow of the adhesive can be suppressed in the radial direction of the bottom face 70b, and the adhesive 72 can be further prevented from flowing into the fitting hole 71.

In the above embodiment, the ferrule is fitted to the sleeve member in a state where the ferrule as the holding member is made to hold the fluorescent body such that the outer periphery of the fluorescent body is covered with the sleeve member. However, the present invention is not limited thereto, and the sleeve member is made to hold the fluorescent body directly.

Additionally, although the electronic endoscope that observes an image obtained by imaging the state of the subject using an imaging element has been described as an example in the above embodiment, the present invention is not limited thereto, and can also be applied to an endoscope that adopts an optical image guide to observe the state of a subject. Furthermore, although the endoscope including the two illumination optical units has been described as an example in the above embodiment, the present invention is not limited thereto, and can be applied also to an endoscope including one illumination optical unit or an endoscope including three or more illumination optical units.

## Claims

1. An illumination optical unit (26A, 26B) for an endoscope comprising:
an optical fiber (37A, 37B) that guides a laser beam supplied from a laser beam source to a distal end of the optical fiber and emits the guided laser beam from the distal end;
a fluorescent body (38) that is excited by the laser beam emitted from the optical fiber to emit florescent light and that forms white light consisting of the fluorescent light and the laser beam;
a sleeve member (61) that covers an outer periphery of the fluorescent body and has a distal end opened; and
a protective cover (36) that is held by the sleeve member, covers the distal end side of the fluorescent body, and transmits the fluorescent light and the laser beam, **characterised in that**
an adhesive (72) having glass beads mixed thereinto is disposed at a gap between the sleeve member and the protective cover to seal the distal end of the sleeve member.

2. The illumination optical unit for an endoscope according to Claim 1,
wherein the sleeve member is formed with a receiving portion consisting of an inner peripheral face that faces an outer peripheral face of the protective cover and a bottom face that intersects the inner peripheral face and faces an end face of the protective cover, and the adhesive is disposed at least at a gap between the outer peripheral face and the inner peripheral face.

3. The illumination optical unit for an endoscope according to Claim 2,
wherein the glass beads to be mixed into the adhesive have a particle size smaller than the distance between the outer peripheral face and the inner peripheral face.

4. The illumination optical unit for an endoscope according to any one of Claims 1 to 3,
wherein the average particle size of the glass beads is 0.4 to 0.7 times the distance between the outer peripheral face and the inner peripheral face.

5. The illumination optical unit for an endoscope according to any one of Claims 1 to 4,
wherein the particle size of the glass beads is equal to or greater than 3.2 µm and less than 20 µm.

6. The illumination optical unit for an endoscope according to any one of Claims 1 to 5,
wherein the bottom face of the sleeve member is formed with a circumferential groove.

7. The illumination optical unit for an endoscope according to any one of Claims 1 to 6, further comprising a holding member having a fluorescent body holding portion that holds the fluorescent body and is opened on the distal end side that faces the protective cover, and a through hole that is continuous with a proximal end of the fluorescent body holding portion and allows the optical fiber to be inserted therethrough,
wherein the holding member is formed in a substantially cylindrical shape that fits to the sleeve member, and the surface of the fluorescent body holding portion is provided with a reflective film that reflects the white light emitted from the fluorescent body.

8. The illumination optical unit for an endoscope according to any one of Claims 1 to 7,
wherein the adhesive is a silicon-based adhesive.

9. The illumination optical unit for an endoscope according to any one of Claims 1 to 7,
wherein the glass beads to be mixed into the adhesive have a light-scattering property.

10. A method of manufacturing an illumination optical unit for an endoscope including: an optical fiber that guides a laser beam supplied from a laser beam source to a distal end of the optical fiber and emits the guided laser beam from the distal end; a fluorescent body that is excited by the laser beam emitted from the optical fiber to emit florescent light and that forms white light including the fluorescent light and the laser beam; a sleeve member that covers an outer periphery of the fluorescent body and has a distal end opened; and a protective cover that is held by the sleeve member, covers the distal end side of the fluorescent body, and transmits the fluorescent light and the laser beam, the method comprising:
disposing an adhesive having glass beads mixed thereinto at least at a gap between the sleeve member and the protective cover, to seal the distal end of the sleeve member with the protective cover;
inserting the fluorescent body and the optical fiber into the inside of the sleeve member from the proximal end side of the sleeve member whose distal end is sealed with the protective cover, to bring the fluorescent body into close contact with the protective cover; and
making the sleeve hold the fluorescent body and the optical fiber in a state where the fluorescent body is brought into close contact with the protective cover, and the optical fiber is arranged on the proximal end side of the fluorescent body.

## Patentansprüche

1. Optische Beleuchtungseinheit (26A, 26B) für ein Endoskop umfassend:
ein optische Faser (37A, 37B), die einen von einer Laserstrahlquelle gelieferten Laserstrahl zu einem distalen Ende der optischen Faser führt und den geführten Laserstrahl von dem distalen Ende emittiert;
ein Fluoreszenzkörper (38), der von dem von der optischen Faser emittierten Laserstahl angeregt wird, um Fluoreszenzlicht zu emittieren, und der Weißlicht bestehend aus dem Fluoreszenzlicht und dem Laserstrahl bildet;
ein Hülsenglied (61), dass eine äußere Peripherie des Fluoreszenzkörpers abdeckt und ein geöffnetes distales Ende aufweist; und
eine Schutzabdeckung (36), die von dem Hülsenglied gehalten wird, die distale Endseite des Fluoreszenzkörpers abdeckt und das Fluoreszenzlicht und den Laserstahl transmittiert,
**dadurch gekennzeichnet, dass**
ein Klebstoff (72) mit darin hineingemischten Glaskügelchen an einer Aussparung zwischen dem Hülsenglied und der Schutzabdeckung angeordnet ist, um das distale Ende des Hülsenglieds abzudichten.

2. Optische Beleuchtungseinheit für ein Endoskop nach Anspruch 1,
wobei das Hülsenglied mit einem Empfangsabschnitt ausgebildet ist, der aus einer inneren Peripheriefläche, die einer äußeren Peripheriefläche der Schutzabdeckung gegenüberliegt, und einer Unterseite, die die innere Peripheriefläche schneidet und einer Endfläche der Schutzabdeckung gegenüberliegt, besteht, und wobei der Klebstoff zumindest an einer Aussparung zwischen der äußeren Peripheriefläche und der inneren Peripheriefläche angeordnet ist.

3. Optische Beleuchtungseinheit für ein Endoskop nach Anspruch 2,
wobei die in den Klebstoff zu mischenden Glaskügelchen eine Partikelgröße kleiner als der Abstand zwischen der äußeren Peripheriefläche und der inneren Peripheriefläche aufweisen.

4. Optische Beleuchtungseinheit für ein Endoskop gemäß einem der Anprüche 1 bis 3,
wobei die durchschnittliche Partikelgröße der Glaskügelchen das 0,4- bis 0,7-fache des Abstands zwischen der äußeren Peripheriefläche und der inneren Peripheriefläche beträgt.

5. Optische Beleuchtungseinheit für ein Endoskop nach einem der Ansprüche 1 bis 4,
wobei die Partikelgröße der Glaskügelchen gleich oder größer als 3,2 µm und weniger als 20 µm ist.

6. Optische Beleuchtungseinheit für ein Endoskop nach einem der Ansprüche 1 bis 5,
wobei die Unterseite des Hülsenglieds mit einer umlaufenden Furche ausgebildet ist.

7. Optische Beleuchtungseinheit für ein Endoskop nach einem der Ansprüche 1 bis 6,
weiterhin umfassend ein Halteglied mit einem Fluoreszenzkörper-Halteabschnitt, der den Fluoreszenzkörper hält und auf der distalen Endseite, die der Schutzabdeckung gegenüberliegt, geöffnet ist und ein Durchgangsloch, das in ein proximale Ende des Fluoreszenzkörper-Halteabschnitts übergeht und es erlaubt, dass die optische Faser dahindurch eingesetzt wird.
wobei das Halteglied in einer im Wesentlichen zylindrischen Form ausgebildet ist, die zu dem Hülsenglied passt und die Oberfläche des Fluoreszenzkörper-Halteabschnitts mit einem reflektierenden Film versehen ist, der das von dem Fluoreszenzkörper emittierte Weißlicht refelktiert.

8. Optische Beleuchtungseinheit für ein Endoskop nach einem der Ansprüche 1 bis 7,
wobei der Klebstoff ein silikonbasierter Klebstoff ist.

9. Optische Beleuchtungseinheit für ein Endoskop nach einem der Ansprüche 1 bis 7,
wobei die in den Klebstoff zu mischenden Glaskügelchen eine Lichtstreueigenschaft aufweisen.

10. Verfahren zur Herstellung einer optische Beleuchtungseinheit für ein Endoskop enthaltend: ein optische Faser, die einen von einer Laserstrahlquelle gelieferten Laserstrahl zu einem distalen Ende der optischen Faser führt und den geführten Laserstrahl von dem distalen Ende emittiert; ein Fluoreszenzkörper, der von dem von der optischen Faser emittierten Laserstahl angeregt wird, um Fluoreszenzlicht zu emittieren, und der Weißlicht enthaltend das Fluoreszenzlicht und den Laserstrahl bildet; ein Hülsenglied, dass eine äußere Peripherie des Fluoreszenzkörpers abdeckt und ein geöffnetes distales Ende aufweist; und eine Schutzabdeckung, die von dem Hülsenglied gehalten wird, die distale Endseite des Fluoreszenzkörpers abdeckt und das Fluoreszenzlicht und den Laserstahl transmittiert, wobei das Verfahren umfasst:
Anordnen eines Klebstoffs mit darin hineingemischten Glaskügelchen zumindest an einer Aussparung zwischen dem Hülsenglied und der Schutzabdeckung, um das distale Ende des Hülsenglieds mit der Schutzabdeckung abzudichten;
Einsetzen des Fluoreszenzkörpers und der optischen Faser in die Innenseite des Hülsenglieds von der proximalen Endseite des Hülsenglieds, dessen distales Ende mit der Schutzabdeckung abgedichtet ist, um den Fluoreszenzkörper in engen Kontakt mit der Schutzabdeckung zu bringen; und
Vorsehen, dass die Hülse den Fluoreszenzkörper und die optische Faser in einem Zustand hält, in dem der Fluoreszenzkörper in engen Kontakt mit der Schutzabdeckung gebracht ist und die optische Faser auf der proximalen Endseite des Fluoreszenzkörpers angeordnet ist.

## Revendications

1. Unité optique d'éclairage (26A, 26B) destinée à un endoscope, comprenant :
une fibre optique (37A, 37B) qui guide un faisceau laser fourni à partir d'une source de faisceau laser à une extrémité distale de la fibre optique et émet le faisceau laser guidé à partir de l'extrémité distale ;
un corps fluorescent (38), qui est excité par le faisceau laser émis à partir de la fibre optique afin d'émettre une lumière fluorescente et qui forme une lumière blanche consistant en la lumière fluorescente et le faisceau laser ;
un élément formant manchon (61) qui couvre une périphérie extérieure du corps fluorescent et présente une extrémité distale ouverte, et
un couvercle protecteur (36), qui est tenu par l'élément formant manchon, couvre le côté d'extrémité distale du corps fluorescent, et transmet la lumière fluorescente et le faisceau laser, **caractérisé en ce que**
un adhésif (72), dans lequel sont mélangées des billes de verre, est disposé au niveau d'un espace entre l'élément formant manchon et le couvercle protecteur afin de sceller l'extrémité distale de l'élément formant manchon.

2. Unité optique d'éclairage destinée à un endoscope selon la revendication 1,
dans laquelle l'élément formant manchon est formé avec une partie de réception consistant en une face périphérique intérieure, laquelle fait face à une face périphérique extérieure du couvercle protecteur et une face inférieure qui coupe la face périphérique intérieure et fait face à une face d'extrémité du couvercle protecteur, et l'adhésif est disposé au moins au niveau d'un espace entre la face périphérique extérieure et la face périphérique intérieure.

3. Unité optique d'éclairage destinée à un endoscope selon la revendication 2,
dans laquelle les billes de verre à mélanger dans l'adhésif présentent une taille de particule inférieure à la distance entre la face périphérique extérieure et la face périphérique intérieure.

4. Unité optique d'éclairage destinée à un endoscope selon l'une quelconque des revendications 1 à 3,
dans laquelle la taille de particule moyenne des billes de verre est égale à 0,4 à 0,7 fois la distance entre la face périphérique extérieure et la face périphérique intérieure.

5. Unité optique d'éclairage destinée à un endoscope selon l'une quelconque des revendications 1 à 4,
dans laquelle la taille de particule des billes de verre est supérieure ou égale à 3,2 µm et inférieure à 20 µm.

6. Unité optique d'éclairage destinée à un endoscope selon l'une quelconque des revendications 1 à 5,
dans laquelle la face inférieure de l'élément formant manchon est formée avec une rainure circonférentielle.

7. Unité optique d'éclairage destinée à un endoscope selon l'une quelconque des revendications 1 à 6,
comprenant en outre un élément de support présentant une partie de support pour corps fluorescent qui tient le corps fluorescent et est ouverte sur le côté d'extrémité distale faisant face au couvercle protecteur, et un trou traversant qui est continu avec une extrémité proximale de la partie de support pour corps fluorescent et permet l'insertion de la fibre optique à travers lui,
dans laquelle l'élément de support est façonné sous une forme sensiblement cylindrique qui s'adapte à l'élément formant manchon, et la surface de la partie de support pour corps fluorescent est dotée d'un film réflecteur qui reflète la lumière blanche émise à partir du corps fluorescent.

8. Unité optique d'éclairage destinée à un endoscope selon l'une quelconque des revendications 1 à 7,
dans laquelle l'adhésif est un adhésif à base de silicone.

9. Unité optique d'éclairage destinée à un endoscope selon l'une quelconque des revendications 1 à 7,
dans laquelle les billes de verre à mélanger avec l'adhésif présentent une propriété de diffusion de la lumière.

10. Procédé de fabrication d'une unité optique d'éclairage destinée à un endoscope incluant :
une fibre optique qui guide un faisceau laser fourni à partir d'une source de faisceau laser à une extrémité distale de la fibre optique et émet le faisceau laser guidé à partir de l'extrémité distale ; un corps fluorescent, qui est excité par le faisceau laser émis à partir de la fibre optique afin d'émettre une lumière fluorescente et qui forme une lumière blanche incluant la lumière fluorescente et le faisceau laser ; un élément formant manchon qui couvre une périphérie extérieure du corps fluorescent et présente une extrémité distale ouverte, et un couvercle protecteur qui est tenu par l'élément formant manchon, couvre le côté d'extrémité distale du corps fluorescent, et transmet la lumière fluorescente et le faisceau laser, le procédé comprenant les étapes pour :
disposer un adhésif, dans lequel sont mélangées des billes de verre, au niveau d'un espace entre l'élément formant manchon et le couvercle protecteur afin de sceller l'extrémité distale de l'élément formant manchon avec le couvercle protecteur ;
insérer le corps fluorescent et la fibre optique dans l'intérieur de l'élément formant manchon à partir du côté d'extrémité proximale de l'élément formant manchon dont l'extrémité distale est scellée avec le couvercle protecteur, afin d'amener le corps fluorescent en contact étroit avec le couvercle protecteur, et
faire en sorte que le manchon tienne le corps fluorescent et la fibre optique dans un état où le corps fluorescent est amené en contact étroit avec le couvercle protecteur, et la fibre optique est agencée sur le côté d'extrémité proximale du corps fluorescent.
